(19) Europäisches Patentamt
European Patent Office
Office européen des brevets

(11) EP 2 691 395 B1

(12) **EUROPEAN PATENT SPECIFICATION**

(45) Date of publication and mention of the grant of the patent:
**30.08.2017 Bulletin 2017/35**

(21) Application number: **12712846.0**

(22) Date of filing: **28.03.2012**

(51) Int Cl.:
***C07D 487/04*** *(2006.01)* ***A61K 31/519*** *(2006.01)*
***A61P 35/00*** *(2006.01)*

(86) International application number:
**PCT/US2012/030938**

(87) International publication number:
**WO 2012/135338 (04.10.2012 Gazette 2012/40)**

(54) **PROCESSES FOR PREPARING TOFACITINIB SALTS**

VERFAHREN ZUR HERSTELLUNG VON SALZEN VON TOFACITINIB

PROCÉDÉS POUR LA PRÉPARATION DE SELS DE TOFACITINIB

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.03.2011 US 201161468246 P**
**17.01.2012 US 201261587237 P**

(43) Date of publication of application:
**05.02.2014 Bulletin 2014/06**

(73) Proprietor: **ratiopharm GmbH**
**89079 Ulm (DE)**

(72) Inventors:
- **Coutable, Ludovic**
  **89077 Ulm (DE)**
- **Albrecht, Wolfgang**
  **89075 Ulm (DE)**
- **Janßen, Christian, Dr.**
  **30161 Hannover (DE)**

(74) Representative: **D Young & Co LLP**
**120 Holborn**
**London EC1N 2DY (GB)**

(56) References cited:
**WO-A1-02/096909** **WO-A1-03/048162**
**WO-A2-2009/034582**

- **JACQUES J ET AL: "Enantiomers, Racemates, and Resolutions, passage", ENANTIOMERS, RACEMATES AND RESOLUTIONS, XX, XX, 1981, pages 252-263,396, XP002292433,**
- **BERGE S M ET AL: "PHARMACEUTICALS SALTS", JOURNAL OF PHARMACEUTICAL SCIENCES, AMERICAN PHARMACEUTICAL ASSOCIATION, WASHINGTON, US, vol. 66, no. 1, 1977, pages 1-19, XP000562636, ISSN: 0022-3549, DOI: 10.1002/JPS.2600660104**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 691 395 B1

## Description

Cross-Reference to Related Applications

This patent application claims the benefits of U.S. Provisional Patent Application Nos. 61/468,246 filed March 28, 2011, and 61/587,237 filed January 17, 2012.

Field of the Invention

The present invention encompasses, methods for the preparation of Tofacitinib acid addition salts.

Background of the Invention

Tofacitinib, 3-{(3R,4R)-4-methyl-3-[methyl-(7H-pyrrolo[2,3-d]pyrimidin-4-yl)-amino]-piperidin-1-yl}3-oxo-propionitrile having the following formula:

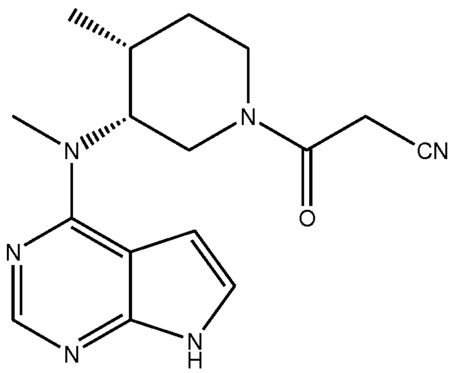

is under investigation as an inhibitor of protein kinases, such as the enzyme Janus Kinase 3 ("JAK3"). It has also been investigated as an immunosuppressive agent for the therapy of several conditions such as organ transplants, xeno transplantation, lupus, multiple sclerosis, rheumatoid arthritis, psoriasis, Type I diabetes and complications from diabetes, cancer, asthma, atopic dermatitis, autoimmune thyroid disorders, ulcerative colitis, Crohn's disease, Alzheimer's disease, leukemia and other indications, where immunosuppression would be desirable (see WO 03/048126).

Tofacitinib, as well as certain pharmaceutically acceptable salts thereof, is described in WO 01/042246 and WO 02/096909. WO 03/048162 describes crystalline and amorphous forms of the mono-citrate salt of Tofacitinib.

Different salts of an active pharmaceutical ingredient may possess different properties. Such variations in the properties of different salts may provide a basis for improving formulation, for example, by facilitating better processing or handling characteristics, improving the dissolution profile, or improving stability and shelf-life. These variations in the properties of different salts may also provide improvements to the final dosage form, for instance, if they serve to improve bioavailability. Different salts of an active pharmaceutical ingredient may also give rise to a variety of polymorphs or crystalline forms, which may in turn provide additional opportunities to assess variations in the properties and characteristics of a solid active pharmaceutical ingredient.

In conclusion, there is a need for the provision of Tofacitinib acid addition salts, processes for their preparation and pharmaceutical compositions thereof.

Summary of the Invention

The present invention provides a process for producing Tofacitinib acid addition salt, in particular, Tofacitinib mono citrate salt. The process comprises:

(a) providing a solution comprising Tofacitinib in the form of the free base;
(b) treating said solution with a first acid, to obtain a first Tofacitinib acid addition salt;
(c) optionally isolating the first Tofacitinib acid addition salt of step (b);
(d) optionally providing a solution of said first Tofacitinib acid addition salt;
(e) treating the salt of step (b), (c) or (d) with a second acid to obtain a reaction mixture comprising a second Tofacitinib acid addition salt; and
(f) optionally isolating said second Tofacitinib acid addition salt from the reaction mixture of step (e);

wherein,
the $pK_a$ of the first acid is higher than the $pK_a$ of the second acid.

Brief Description of the Drawings

Figure 1 shows an $^1$H-NMR spectrum of Tofacitinib acetate.

Figure 2 shows an IR spectrum of Tofacitinib acetate.

Figure 3 shows an X-ray powder diffractogram of Tofacitinib acetate.

Figure 4 shows an $^1$H-NMR spectrum of Tofacitinib mono-citrate.

Figure 5 shows an IR spectrum of amorphous Tofacitinib mono-citrate.

Figure 6 shows an X-ray powder diffractogram of a solid dispersion of Tofacitinib mono-citrate and amorphous magnesium alumino-metasilicate.

Figure 7 shows an X-ray powder diffractogram of a solid dispersion of Tofacitinib mono-citrate and polyvinyl pyrrolidone.

Figure 8 shows an X-ray powder diffractogram of a solid dispersion of Tofacitinib mono-citrate and microcrystalline cellulose.

Detailed Description of the Invention

The present invention encompasses processes for preparing Tofacitinib acid addition salts in high yield and purity. In some embodiments, salts and solid state forms of Tofacitinib are substantially free of any other salts, polymorphic forms, or of specified polymorphic forms of Tofacitinib, respectively. By "substantially free" or "polymorphically pure", unless indicated otherwise, means that the forms contain 20% (w/w) or less, 10% (w/w) or less, 5% (w/w) or less, 2% (w/w) or less, particularly 1% (w/w) or less, more particularly 0.5% (w/w) or less, and most particularly 0.2% (w/w) or less of any other salts, polymorphs or of a specified polymorph of Tofacitinib. In other embodiments, the acid addition salts and solid state forms of Tofacitinib contain from 1% to 20% (w/w), from 5% to 20% (w/w), or from 5% to 10% (w/w) of any other salts, solid state form or of a specified polymorph of Tofacitinib.

As used herein, the term "isolated" in reference to any of Tofacitinib salts or solid state forms thereof of the present invention corresponds to Tofacitinib salt or solid state form thereof that is physically separated from the reaction mixture, where it is formed.

As used herein, unless stated otherwise, the XRPD measurements are taken using copper K$\alpha$ radiation wavelength 1.5406 Å.

A thing, *e.g.*, a reaction mixture, may be characterized herein as being at, or allowed to come to "room temperature, often abbreviated "RT." This means that the temperature of the thing is close to, or the same as, that of the space, *e.g.*, the room or fume hood, in which the thing is located. Typically, room temperature is from about 20°C to about 30°C, or about 22°C to about 27°C, or about 25°C.

A process or step may be referred to herein as being carried out "overnight." This refers to a time interval, *e.g.,* for the process or step, that spans the time during the night, when that process or step may not be actively observed. This time interval is from about 8 to about 20 hours, or about 10-18 hours, typically about 16 hours.

As used herein, the term "reduced pressure" refers to a pressure of about 10 mbar to about 50 mbar.

As used herein, the terms "vol." or "volume" can be used to refer to mL per gram of the corresponding Tofacitinib. For example, a statement that 0.5 g of Tofacitinib is dissolved in ten volumes of a Solvent X would be understood to mean that the 0.5 g of Tofacitinib was dissolved in 5 mL of Solvent X.

In the present patent application, a solid state form may be referred to herein as being characterized by graphical data "as depicted in" a Figure. Such data include, for example, powder X-ray diffractograms and solid state NMR spectra. The skilled person will understand that such graphical representations of data may be subject to small variations, e.g., in peak relative intensities and peak positions due to factors such as variations in instrument response and variations in sample concentration and purity, which factors are well known to the skilled person. Nonetheless, the skilled person would readily be capable of comparing the graphical data in the Figures herein with graphical data generated for an

unknown crystal form and confirming whether the two sets of graphical data characterize the same solid state form or two different solid state forms. The skilled person would understand that a solid state form referred to herein as being characterized by graphical data "as shown in" a Figure would include any solid state form of the same chemical characterized by graphical data substantially similar to the Figure except for such small variations, the potential occurrence of which is well known to the skilled person.

A solid state form may be referred to herein as being characterized by data selected from two or more different data groupings, for example, by a powder XRD pattern having a group of specific peaks; or by a powder XRD pattern as shown in a figure depicting a diffractogram, or by "a combination thereof" (or "combinations thereof," or "any combination thereof"), These expressions, e.g., "any combination thereof" contemplate that the skilled person may characterize a crystal form using a combination of characteristic analytical data. For example, the skilled person may characterize a crystal form using a group of four or five characteristic powder XRD peaks, and supplement that characterization with an additional feature observed in the powder X-ray diffractogram, e.g., an additional peak, a characteristic peak shape, a peak intensity, or even the absence of a peak at some position in the powder XRD pattern.

Tofacitinib acetate may be characterized by an $^1$H-NMR spectrum substantially as depicted in Figure 1. Typically, the Tofacitinib acetate may be in an amorphous form.

Tofacitinib acetate salt can be characterized by data selected from: an X-ray powder diffraction substantially as depicted in figure 3; an IR spectrum substantially as depicted in Figure 2; an IR spectrum substantially as depicted in Figure 5; an IR spectrum having peaks at 3230, 3120, 2958, 1711, 1648, 1560, 1489, 1453, 1409, 1366, 1341, 1306, 1277, 1250, 1227, 1049, 925 and 726 $cm^{-1} \pm 1\ cm^{-1}$; and combinations thereof.

Tofacitinib mono-citrate may be characterized by an $^1$H-NMR spectrum substantially as depicted in Figure 4. Amorphous Tofacitinib mono-citrate can be characterized by data selected from: an IR spectrum substantially as depicted in Figure 5; an IR spectrum having peaks at 3119, 2955, 1720, 1618, 1600, 1535, 1450, 1409, 1373, 1348, 1225, 1183, 1052, 904 and 732 $cm^{-1} \pm 1\ cm^{-1}$; and combinations thereof.

The Tofacitinib acid addition salts and solid state forms of the present invention can be used for the preparation of a second Tofacitinib acid addition salt, in particular the mono-citrate, as well as solid state forms and/or formulations thereof.

The Tofacitinib acid addition salt, e.g. Tofacitinib acetate, may be used to prepare a second Tofacitinib acid addition salt, e.g., Tofacitinib mono citrate. The present invention provides a method of manufacturing Tofacitinib acid addition salts, in particular Tofacitinib mono citrate, comprising:

(a) providing a solution comprising Tofacitinib in form of the free base;
(b) treating said solution with a first acid, to obtain a first Tofacitinib acid addition salt;
(c) optionally isolating the first Tofacitinib acid addition salt of step (b);
(d) optionally providing a solution of said first Tofacitinib acid addition salt;
(e) treating the salt of step (b), (c) or (d) with the second acid to obtain a second Tofacitinib acid addition salt; and
(f) optionally isolating said second Tofacitinib acid addition salt from the reaction mixture of step (e);

wherein, the $pK_a$ of the first acid is higher than the $pK_a$ of a second acid.

In step (a), Tofacitinib free base (which can also be synonymously designated as Tofacitinib base, in the form of the freebase or in its basic form) is dissolved in an appropriate solvent. The appropriate solvent may include a $C_1$-$C_5$ alcohol, a $C_3$-$C_7$ ketone, e.g. acetone, a $C_4$-$C_8$ cyclic or aliphatic ether, e.g. tetrahydrofuran (THF), dioxane or diisopropylether, a nitrile, e.g., acetonitrile, a $C_2$-$C_8$ ester e.g., ethyl acetate, or a mixture thereof. Alternatively, the solvent is a mixture of water and one or more of the appropriate solvents mentioned above. For example, the solvent may be methanol. Generally, Tofacitinib free base may be dissolved completely.

In step (b), the solution of step (a) is treated with a first acid having a $pK_a$, which is higher than the $pK_a$ of a second acid, such as citric acid, to obtain a first Tofacitinib acid addition salt. This step may include contacting or reacting the solution of step (a) with the first acid. The treating can be in any suitable order, i.e., the solution of Tofacitinib free base can be added to the first acid, or the first acid can be added to the solution of Tofacitinib free base, as desired. Thus, the first acid can be provided as a solution, wherein, in this process, the term solution includes a slurry, or in liquid form or solid form and, thus, can also be provided in the absence of any solvent.

The term $pK_a$ refers to the acid dissociation constant. Generally, an acid dissociation constant, $K_a$, (also known as acidity constant, or acid-ionization constant) is a quantitative measure of the strength of an acid in solution. It is the equilibrium constant for a chemical reaction, known as dissociation in the context of acid-base reactions. The equilibrium can be written symbolically as:

$$HA \rightleftharpoons A^- + H^+$$

where HA is a generic acid that dissociates by splitting into $A^-$, known as the conjugate base of the acid, and the hydrogen ion or proton, $H^+$, which, in the case of aqueous solutions, exists as a solvated hydronium ion. The chemical species HA, A- and $H^+$ are said to be in equilibrium when their concentrations do not change with the passing of time. The dissociation constant is usually written as a quotient of the equilibrium concentrations (in mol/L), denoted by [HA], $[A^-]$ and $[H^+]$:

$$K_a = \frac{[A^-][H^+]}{[HA]}$$

Due to the orders of magnitude spanned by $K_a$ values, the logarithmic term $pK_a$ is equal to $-\log_{10} K_a$, and is referred to in the context of the subject invention as the acid dissociation constant:

$$pK_a = -\log_{10} K_a$$

The larger the value of $pK_a$, the smaller the extent of dissociation.

The first acid has a $pK_a$ which is higher than the $pK_a$ of the second acid. For example, when the second acid is citric acid, the first acid would be an acid having $pK_a$ higher than 3.09 (which is the reported $pK_{a1}$ of citric acid)).

According to some embodiments, the first acid is selected from acetic acid ($pK_a$ about 4.76), formic acid ($pK_a$ about 3.75), propanoic acid ($pK_a$ about 4.86), 3-hydroxypropanoic acid ($pK_a$ about 4.51), succinic acid ($pK_a$ about 4.16), butanoic acid ($pK_a$ about 4.83), 2-methyl-propanoic or isobutyric acid ($pK_a$ about 4.88), 3-hydroxybutanoic acid (pKa about 4.70), 4-hydroxybutanoic acid ($pK_a$ about 4.72), uric acid ($pK_a$ about 3.89), glutaric acid ($pK_a$ about 4.31), methylsuccinic acid ($pK_a$ about 4.13), pentanoic acid ($pK_a$ about 4.84), trimethylacetic acid ($pK_a$ about 5.03), ascorbic acid ($pK_a$ about 4.10), hexanoic or caproic acid ($pK_a$ about 4.85), 4-methylpentanoic acid ($pK_a$ about 4.84), benzoic acid ($pK_a$ about 4.19), *m*-hydroxybenzoic acid ($pK_a$ about 4.06), *p*-hydroxybenzoic acid ($pK_a$ about 4.48), cyclohexanecarboxylic acid ($pK_a$ about 4.90), phenylacetic acid ($pK_a$ about 4.28), *cis*-cinnamic acid ($pK_a$ about 3.89), *trans*-cinnamic acid ($pK_a$ about 4.44), hippuric acid ($pK_a$ about 3.55), D-gluconic acid ($pK_a$ about 3.76), DL-lactic acid ($pK_a$ about 3.86), oleic acid ($pK_a$ about 4), 4-acetamidobenzoic acid ($pK_a$ about 4.3), adipic acid ($pK_a$ about 4.44), sebacic acid (pKa about 4.59), (+)-camphoric acid ($pK_a$ about 4.72), nicotinic acid ($pK_a$ about 4.85), capric or decanoic acid ($pK_a$ about 4.9), lauric acid ($pK_a$ about 4.9), palmitic acid ($pK_a$ about 4.9), stearic acid ($pK_a$ about 4.9), undecylenic acid ($pK_a$ about 4.9), caprylic or octanoic acid (pKa about 4.91), orotic acid ($pK_a$ about 5.85), and carbonic acid ($pk_a$ about 6.46). In a preferred embodiment the acid may be selected from acetic acid, formic acid, and propionic acid. For example, the first acid may be acetic acid.

According to some embodiments, the first acid is liquid at a temperature of about 20 °C and a pressure of about 1013 mbar. Those acids include, for example, acetic acid and formic acid. Moreover, the first acid typically has a boiling point such as of about 30°C to about 200°C, or of about 50°C to about 150 °C, at a pressure of about 1013 mbar.

In a preferred embodiment, the first acid is freely miscible with water. The term "freely miscible," unless indicated otherwise, means that that any amount of the first acid can be mixed with any amount of water without forming separate phases, thus, without forming a dispersion or emulsion. Alternatively, the first acid may have solubility in water such as of about 10 g/l to about 10,000 g/l at about 20 °C, or of about 50 g/l to about 1,000 g/l at a temperature of about 20 °C and a pressure of about 1013 mbar.

The first acid may be added in equivalent molar amounts to the solution of the Tofacitinib free base. Alternatively, the first acid can be added in excess. According to some embodiments, the first acid is added in a molar ratio such as of about 0.2 to 2, or of about 0.5 to 1.5, or of about 0.8 to 1.2, for example, of about 1 to 1, to the solution of the Tofacitinib free base.

As mentioned above, the first Tofacitinib acid addition salt obtained in step (b) may be isolated, according to step (c), for example, by filtration. The isolation step (c) can further include one or more purification steps for purifying the first Tofacitinib acid addition salt. Isolation and/or purification can be achieved by performing one or more steps such as, e.g. precipitation, resolution or crystallization.

When the Tofacitinib first acid addition salt is isolated, it may then be dissolved in an appropriate solvent according to step (d). An appropriate solvent is a solvent suitable for dissolving the first Tofacitinib acid addition salt. Thus, choice of the solvent usually depends on the first acid used in step (b). Appropriate solvents include the solvents as listed above for step (a). Thus, the first Tofacitinib acid addition salt can be dissolved for example in water or an organic solvent, such as a $C_1$-$C_5$ alcohol, or a mixture of solvents, or a mixture comprising water and one or more organic solvents. For example, the solvent may be methanol.

The solution comprising the first acid addition salt is treated with a second acid, for example, with citric acid, according to step (e), to obtain a second acid addition salt of Tofacitinib, for example, Tofacitinib mono citrate (which can also be synonymously designated as Tofacitinib hemicitrate). This step, which typically includes contacting or reacting the solution comprising the first acid addition salt with the second acid, can be in any suitable order: i.e., the solution of the Tofacitinib acid addition salt can be added to the second acid such as citric acid, or the second acid can be added to the solution of the Tofacitinib acid addition salt, as desired. The second acid can be added as a solution, as a slurry, or in solid form, for example, as a solution.

In a preferred embodiment, the crude reaction product of step (b), which is the first acid addition salt of Tofacitinib is used directly in step (e), i.e. in a one-pot manner.

The second acid, such as citric acid may be added in equivalent molar amounts to the solution of the first Tofacitinib acid addition salt. Alternatively, the second acid, such as citric acid can be added in excess. According to some embodiments, the second acid is added in a molar ratio such as of about 0.2 to 2, or of about 0.5 to 1.5, or of about 0.8 to 1.2, for example, of about 1 to 1, to the solution of the first Tofacitinib acid addition salt.

In the optional step (f), of the method of the invention, the second acid addition salt of Tofacitinib, for example Tofacitinib mono citrate, can optionally be isolated from the reaction mixture of step (e), for example, by filtration. The isolation step (f) can further include one or more purification steps for purifying the second Tofacitinib acid addition salt. Isolation and/or purification can be achieved by performing one or more steps such as, e.g., precipitation, resolution or (re)crystallization.

Further features of the present invention are set out in the following numbered clauses.

Clause 1. Method of manufacturing a Tofacitinib acid addition salt, such as Tofacitinib mono citrate, comprising the steps of

(a) providing a solution comprising Tofacitinib free base,
(b) treating said solution with a first acid having a $pK_a$, which is higher than the $pK_a$ of a second acid, in particular higher than the $pK_a$ of citric acid, to obtain a first Tofacitinib acid addition salt,
(c) optionally isolating the first Tofacitinib acid addition salt of step (b),
(d) optionally providing a solution of said first Tofacitinib acid addition salt,
(e) treating the salt of step (b), (c) or (d) with the second acid, particularly, with citric acid to obtain a Tofacitinib second acid addition salt, such as Tofacitinib mono citrate, and
(f) optionally isolating said Tofacitinib second acid addition salt from the reaction mixture of step (e).

Clause 2. Method of manufacturing a Tofacitinib acid addition salt, particularly, Tofacitinib mono citrate according to Clause 1, wherein in step (a) Tofacitinib in form of the free base is dissolved in water and/or an organic solvent, preferably a $C_1$-$C_5$ alcohol, most preferably methanol.

Clause 3. Method of manufacturing a second acid addition salt, particularly, Tofacitinib mono citrate according to Clause 1 or 2, wherein the first acid is selected from acetic acid, formic acid, propanoic acid, 3- hydroxypropanoic acid, succinic acid, butanoic acid, 2-methylpropanoic acid, 3- hydroxybutanoic acid, 4-hydroxybutanoic acid, uric acid, glutaric acid, methylsuccinic acid, pentanoic acid, trimethylacetic acid, ascorbic acid, hexanoic acid, 4-methylpentanoic acid, benzoic acid, *m*-hydroxybenzoic acid, *p*-hydroxybenzoic acid, cyclohexanecarboxylic acid, phenylacetic acid, *cis*-cinnamic acid, *trans*-cinnamic acid, hippuric acid, D-gluconic acid, D,L-lactic acid, oleic acid, 4-acetamidobenzoic acid, adipic acid, sebacic acid, (+)-camphoric acid, nicotinic acid, capric or decanoic acid, lauric acid, palmitic acid, stearic acid, undecylenic acid, caprylic or octanoic acid, orotic acid, and carbonic acid, preferably acetic acid.

Clause 4. Method of manufacturing a Tofacitinib second acid addition salt, particularly, Tofacitinib mono citrate according to any one of Clauses 1 to 3, wherein the first acid is freely miscible with water or has a solubility in water of 10 g/l to 10,000 g/l at 20 °C, preferably of 50 g/l to 1,000 g/l at a temperature of 20 °C and a pressure of 1013 mbar.

Clause 5. Method of manufacturing a Tofacitinib second acid addition salt, particularly, Tofacitinib mono citrate according to any one of Clauses 1 to 4, wherein the first acid is liquid at a temperature of 20 °C and a pressure of 1013 mbar.

Clause 6. Method of manufacturing a Tofacitinib second acid addition salt, particularly, Tofacitinib mono citrate according to any one of Clauses 1 to 5, wherein the first acid has a boiling point of 30 to 200 °C, preferably of 50 to 150 °C at a pressure of 1013 mbar.

Clause 7. Method of manufacturing Tofacitinib second acid addition salt, particularly, Tofacitinib mono citrate according to any one of Clauses 1 to 6, wherein in step (d) the first Tofacitinib acid addition salt is dissolved in water or an organic solvent, preferably a $C_1$-$C_5$ alcohol, most preferably methanol.

The Examples are set forth to aid in understanding the invention but are not intended to, and should not be construed to limit its scope in any way.

X-Ray Powder Diffraction

The samples were analyzed on a D8 Advance X-ray powder diffractometer (Bruker-AXS, Karlsruhe, Germany). The sample holder was rotated in a plane parallel to its surface at 20 rpm during the measurement. Further conditions for the measurements are summarized below. The raw data were analyzed with the program EVA (Bruker-AXS, Germany).

| | standard measurement |
|---|---|
| radiation | Cu $K_\alpha$ ($\lambda$ = 1.5406 Å) |
| source | 38 kV / 40 mA |
| detector | Vantec |
| detector slit | variable |
| divergence slit | v6 |
| antiscattering slit | v6 |
| $2\theta$ rage /° | $2 \leq 2\theta \leq 55$ |
| step size /° | 0.017 |

Nuclear magnetic resonance (NMR) spectroscopy

Instrument: Varian Mercury 400 Plus NMR Spectrometer, Oxford AS, 400 MHz

Infrared Spectroscopy

| | |
|---|---|
| Instrument: | Thermo Nicolet, Avatar 330 FT-IR. Smart Endurance Diamond-ATR |
| Software : | Omnic Vers. 6.1a |

EXAMPLES

Reference examples:

1. The crystalline Tofacitinib mono-citrate can be prepared by reacting methyl-[(3R, 4R)-4-methyl-piperidin-3-yl)]-(7H-pyrrolo[2,3-d]pyrimidin-4-yl)-amine with methyl cyanoacetate according to the method described in lit. Org. Lett., Vol. 11, No. 9, 2009.

2. The Tofacitinib free base can be prepared according to the following process: The obtained Tofacitinib mono-citrate was reacted with a solution of potassium carbonate 10%, followed by extraction with dichloromethane. The resulting Tofacitinib free-base was obtained as an amorphous off-white powder in high purity (>99%).

Examples marked with an asterisk (*) are provided by way of reference only.

Example 1: Preparation of amorphous Tofacitinib mono-citrate

Step1: Preparation of Tofacitinib mono-acetate

Tofacitinib free-base (2.50 g) was dissolved in methanol (32 ml). Acetic acid (0.55 ml) was added drop-wise at room temperature and the resulting solution was stirred at room temperature for 1 hour. The mixture was then concentrated and the residual off-white solid was dried in vacuo to yield amorphous Tofacitinib mono-acetate, which was characterized by $^1$H-NMR, IR-spectroscopy and XRPD.

Step2: Preparation of amorphous Tofacitinib mono-citate

Amorphous Tofacitinib mono-acetate (0.50 g) was dissolved in water (25 ml) at 40°C. The solution was then cooled to room temperature, and anhydrous citric acid (0.258 g) was added portionwise and the resulting solution was stirred at room temperature for 1 hour. The mixture was then lyophilized to yield amorphous Tofacitinib mono-citrate which was dried at 75°C/20 mbar for 1 day.

Example 2*: Preparation of amorphous Tofacitinib mono-citrate

Tofacitinib free-base (1.00 g) and anhydrous citric acid (0.615 g) were introduced into a cylindric zirconia jar (internal volume 80 ml) along with 12 zirconia balls (outer diameter 10 mm). The milling operation was performed with a planetary mill PULVERISETTE 6 at a rotation speed of 400 rpm for 1 hour. White powder was obtained.

Example 3*: Preparation of amorphous Tofacitinib mono-citrate

Crystalline Tofacitinib mono-citrate (0.20 g) was dissolved in water (20 ml) at 75°C. The solution was cooled to room temperature, and lyophilized to yield amorphous Tofacitinib mono-citrate.

Example 4*: Preparation of amorphous Tofacitinib mono-citrate as solid dispersion with amorphous magnesium alumino-metasilicate

Crystalline Tofacitinib mono-citrate (1.0 g) and amorphous magnesium alumino-metasilicate (Neusilin® US2) (1.0 g) were introduced into a cylindric zirconia jar (internal volume 80 ml) along with 12 zirconia balls (outer diameter 10 mm). The milling operation was performed with a planetary mill PULVERISETTE 6 at a rotation speed of 400 rpm for 1 hour.

Example 5*: Preparation of amorphous Tofacitinib mono-citrate as solid dispersion with amorphous magnesium alumino-metasilicate

Crystalline Tofacitinib mono-citrate (1.33 g) and amorphous magnesium alumino-metasilicate (Neusilin® US2) (0.67 g) were introduced into a cylindric zirconia jar (internal volume 80 ml) along with 12 zirconia balls (outer diameter 10 mm). The milling operation was performed with a planetary mill PULVERISETTE 6 at a rotation speed of 400 rpm for 1 hour.

Example 6*: Preparation of Tofacitinib mono-citrate as solid dispersion with polyvinyl pyrrolidone

Crystalline Tofacitinib mono-citrate (1.0 g) and polyvinyl pyrrolidone (Kollidon® 30) (1.0 g) were introduced into a cylindric zirconia jar (internal volume 80 ml) along with 12 zirconia balls (outer diameter 10 mm). The milling operation was performed with a planetary mill PULVERISETTE 6 at a rotation speed of 400 rpm for 1 hour.

Example 7*: Preparation of Tofacitinib mono-citrate as solid dispersion with microcrystalline cellulose

Crystalline Tofacitinib mono-citrate (1.0 g) and microcrystalline cellulose (Avicel® PH-102) (1.0 g) were introduced into a cylindric zirconia jar (internal volume 80 ml) along with 12 zirconia balls (outer diameter 10 mm). The milling operation was performed with a planetary mill PULVERISETTE 6 at a rotation speed of 400 rpm for 1 hour.

Example 8: Preparation of Tofacitinib acetate

Tofacitinib free base (1 eq) is dissolved in MeOH (50 volumes). Acetic acid (1 eq) is added and the solution is

stirred at room temperature for 1 h. The volatiles are removed in vacuo and the residual solid is dried in vacuo to obtain Tofacitinib acetate in quantitative yield.

Example 9: Preparation of amorphous Tofacitinib citrate from solution

Tofacitinib acetate (1 eq) is dissolved in MeOH (50 volumes) and citric acid (1 eq) is added to the solution. The mixture is heated to reflux temperature and the volatiles are distilled off. The solid residue is dried under vacuum to obtain Tofacitinib citrate in quantitative yield.

Example 10: Preparation of Tofacitinib citrate by precipitation

A solution of Tofacitinib acetate (1 eq) is dissolved in methanol (50 volumes). Citric acid (1 eq) is added to the reaction mixture and the resulting mixture is stirred at room temperature for 5 min. Diisopropylether (100 volumes) is added in one portion. The resulting solid is removed by filtration immediately and then dried in vacuo.

Example 11: Preparation of amorphous Tofacitinib citrate by spray-drying

A solution of Tofacitinib acetate (1 eq) is dissolved in methanol (50 volumes). Citric acid (1 eq) is added to the reaction mixture and the resulting mixture is subjected to spray-drying to yield amorphous Tofacitinib citrate.

Example 12: Preparation of amorphous Tofacitinib citrate by lyophilisation

A solution of Tofacitinib acetate (1 eq) is dissolved in methanol (50 volumes). Citric acid (1 eq) is added to the reaction mixture and the resulting solution is dried on a freeze dryer to yield amorphous Tofacitinib citrate in quantitative yield.

Example 13: Preparation of amorphous Tofacitinib citrate by melt processing

Tofacitinib acetate (1 eq) and citric acid (1 eq) are mixed and the resulting mixture is blended with polyoxyethylene polyoxypropylene copolymer (molecular weight approximately 8350) in a ratio of 1:1 w/w. The resulting blend is melt extruded at a temperature from 80 to 120 °C and subsequently granulated.

Example 14: Preparation of amorphous Tofacitinib citrate by co-precipitation

A solution of Tofacitinib acetate (1 eq) and polyvinylpyrrolidone (1eq) are dissolved in methanol (50 volumes). Citric acid (1 eq) is added to the reaction mixture and the resulting mixture is stirred at room temperature for 5 min. Diisopropylether (100 volumes) is added in one portion. The resulting solid is removed by filtration immediately and then dried in vacuo to yield amorphous Tofacitinib citrate in quantitative yield.

## Claims

**1.** A process for producing a Tofacitinib acid addition salt, said process comprising:

(a) providing a solution comprising Tofacitinib in the form of the free base;
(b) treating said solution with a first acid, to obtain a first Tofacitinib acid addition salt;
(c) optionally isolating the first Tofacitinib acid addition salt of step (b);
(d) optionally providing a solution of said first Tofacitinib acid addition salt;
(e) treating the salt of step (b), (c) or (d) with a second acid to obtain a reaction mixture comprising a second Tofacitinib acid addition salt; and
(f) optionally isolating said second Tofacitinib acid addition salt from the reaction mixture of step (e);

wherein, the $pK_a$ of the first acid is higher than the $pK_a$ of the second acid.

**2.** The process according to claim 1, wherein the first acid is selected from: acetic acid, formic acid, propanoic acid, 3-hydroxypropanoic acid, succinic acid, butanoic acid, 2-methyl-propanoic acid, 3-hydroxybutanoic acid, 4-hydroxybutanoic acid, uric acid, glutaric acid, methylsuccinic acid, pentanoic acid, trimethylacetic acid, ascorbic acid, hexanoic acid, 4-methylpentanoic acid, benzoic acid, *m*-hydroxybenzoic acid, *p*-hydroxybenzoic acid, cyclohexanecarboxylic

acid, phenylacetic acid, *cis*-cinnamic acid, *trans*-cinnamic acid, hippuric acid, D-gluconic acid, DL-lactic acid, oleic acid, 4-acetamidobenzoic acid, adipic acid, sebacic acid, (+)-camphoric acid, nicotinic acid, decanoic acid, lauric acid, palmitic acid, stearic acid, undecylenic acid, caprylic or octanoic acid, orotic acid, and carbonic acid.

**3.** The process according to claim 2, herein the Tofacitinib first acid is acetic acid.

**4.** The process according to claim 1, wherein the second Tofacitinib acid addition salt is Tofacitinib citrate.

**5.** The process according to claim 1, wherein the solution in step (a) comprises the Tofacitinib base dissolved in a solvent selected from: a $C_1$-$C_5$ alcohol, a $C_3$-$C_7$ ketone, a $C_4$-$C_8$ cyclic or aliphatic ether, a nitrile, a $C_2$-$C_8$ ester, a mixture thereof, and a mixture thereof and water.

**Patentansprüche**

**1.** Verfahren zur Herstellung eines Tofacitinib-Säureadditionssalzes, wobei das Verfahren Folgendes umfasst:

(a) Bereitstellen einer Lösung, die Tofacitinib in Form der freien Base umfasst;
(b) Behandeln der Lösung mit einer ersten Säure, wodurch man zu einem ersten Tofacitinib-Säureadditionssalz gelangt;
(c) gegebenenfalls Isolieren des ersten Tofacitinib-Säureadditionssalzes von Schritt (b) ;
(d) gegebenenfalls Bereitstellen einer Lösung des ersten Tofacitinib-Säureadditionssalzes;
(e) Behandeln des Salzes von Schritt (b), (c) oder (d) mit einer zweiten Säure, wodurch man zu einem Reaktionsansatz, der ein zweites Tofacitinib-Säureadditionssalz umfasst, gelangt; und
(f) gegebenenfalls Isolieren des zweiten Tofacitinib-Säureadditionssalzes von dem Reaktionsansatz von Schritt (e);

wobei der $pK_a$-Wert der ersten Säure höher als der $pK_a$-Wert der zweiten Säure ist.

**2.** Verfahren nach Anspruch 1, wobei die erste Säure aus den Folgenden ausgewählt ist: Essigsäure, Ameisensäure, Propansäure, 3-Hydroxypropansäure, Bernsteinsäure, Butansäure, 2-Methylpropansäure, 3-Hydroxybutansäure, 4-Hydroxybutansäure, Harnsäure, Glutarsäure, Methylbernsteinsäure, Pentansäure, Trimethylessigsäure, Ascorbinsäure, Hexansäure, 4-Methylpentansäure, Benzoesäure, *m*-Hydroxybenzoesäure, *p*-Hydroxybenzoesäure, Cyclohexancarbonsäure, Phenylessigsäure, *cis*-Zimtsäure, *trans*-Zimtsäure, Hippursäure, D-Glukonsäure, DL-Milchsäure, Ölsäure, 4-Acetamidobenzoesäure, Adipinsäure, Sebacinsäure, (+)-Kampfersäure, Nikotinsäure, Decansäure, Laurinsäure, Palmitinsäure, Stearinsäure, Undecylensäure, Caprylsäure oder Octansäure, Orotsäure und Kohlensäure.

**3.** Verfahren nach Anspruch 2, wobei es sich bei der ersten Tofacitinib-Säure um Essigsäure handelt.

**4.** Verfahren nach Anspruch 1, wobei es sich bei dem zweiten Tofacitinib-Säureadditionssalz um Tofacitinib-Citrat handelt.

**5.** Verfahren nach Anspruch 1, wobei die Lösung in Schritt (a) die Tofacitinib-Base gelöst in einem Lösungsmittel, ausgewählt aus den Folgenden, umfasst: einem $C_1$-$C_5$-Alkohol, einem $C_3$-$C_7$-Keton, einem cyclischen oder aliphatischen $C_4$-$C_8$-Ether, einem Nitril, einem $C_2$-$C_8$-Ester, einer Mischung davon, und einer Mischung davon und Wasser.

**Revendications**

**1.** Procédé de production d'un sel d'addition d'acide de tofacitinib, ledit procédé comprenant :

(a) la fourniture d'une solution comprenant du tofacitinib sous la forme de la base libre ;
(b) le traitement de ladite solution par un premier acide, afin d'obtenir un premier sel d'addition d'acide de tofacitinib ;
(c) éventuellement, l'isolement du premier sel d'addition d'acide de tofacitinib issu de l'étape (b) ;
(d) éventuellement, la fourniture d'une solution dudit premier sel d'addition d'acide de tofacitinib ;
(e) le traitement du sel de l'étape (b), (c) ou (d) par un deuxième acide, afin d'obtenir un mélange réactionnel

comprenant un deuxième sel d'addition d'acide de tofacitinib ; et
(f) éventuellement l'isolement dudit deuxième sel d'addition d'acide de tofacitinib à partir du mélange réactionnel de l'étape (e) ;

où le $pK_a$ du premier acide est supérieur au $pK_a$ du deuxième acide.

**2.** Procédé selon la revendication 1, dans lequel le premier acide est choisi parmi : l'acide acétique, l'acide formique, l'acide propanoïque, l'acide 3-hydroxypropanoïque, l'acide succinique, l'acide butanoïque, l'acide 2-méthylpropanoïque, l'acide 3-hydroxybutanoïque, l'acide 4-hydroxybutanoïque, l'acide urique, l'acide glutarique, l'acide méthylsuccinique, l'acide pentanoïque, l'acide triméthylacétique, l'acide ascorbique, l'acide hexanoïque, l'acide 4-méthylpentanoïque, l'acide benzoïque, l'acide m-hydroxybenzoïque, l'acide p-hydroxybenzoïque, l'acide cyclohexanecarboxylique, l'acide phénylacétique, l'acide cis-cinnamique, l'acide trans-cinnamique, l'acide hippurique, l'acide D-gluconique, l'acide DL-lactique, l'acide oléique, l'acide 4-acétamidobenzoïque, l'acide adipique, l'acide sébacique, l'acide (+)-camphorique, l'acide nicotinique, l'acide décanoïque, l'acide laurique, l'acide palmitique, l'acide stéarique, l'acide undécylénique, l'acide caprylique ou octanoïque, l'acide orotique, et l'acide carbonique.

**3.** Procédé selon la revendication 2, dans lequel le premier acide de tofacitinib est l'acide acétique.

**4.** Procédé selon la revendication 1, dans lequel le deuxième sel d'addition d'acide de tofacitinib est le citrate de tofacitinib.

**5.** Procédé selon la revendication 1, dans lequel la solution dans l'étape (a) comprend la base de tofacitinib solubilisée dans un solvant choisi parmi : un alcool en $C_1$-$C_5$, une cétone en $C_3$-$C_7$, un éther aliphatique ou cyclique en $C_4$-$C_8$, un nitrile, un ester en $C_2$-$C_8$, un mélange de ceux-ci, et un mélange de ceux-ci avec de l'eau.

A characteristic $^1$H-NMR spectrum of Tofacitinib mono-acetate

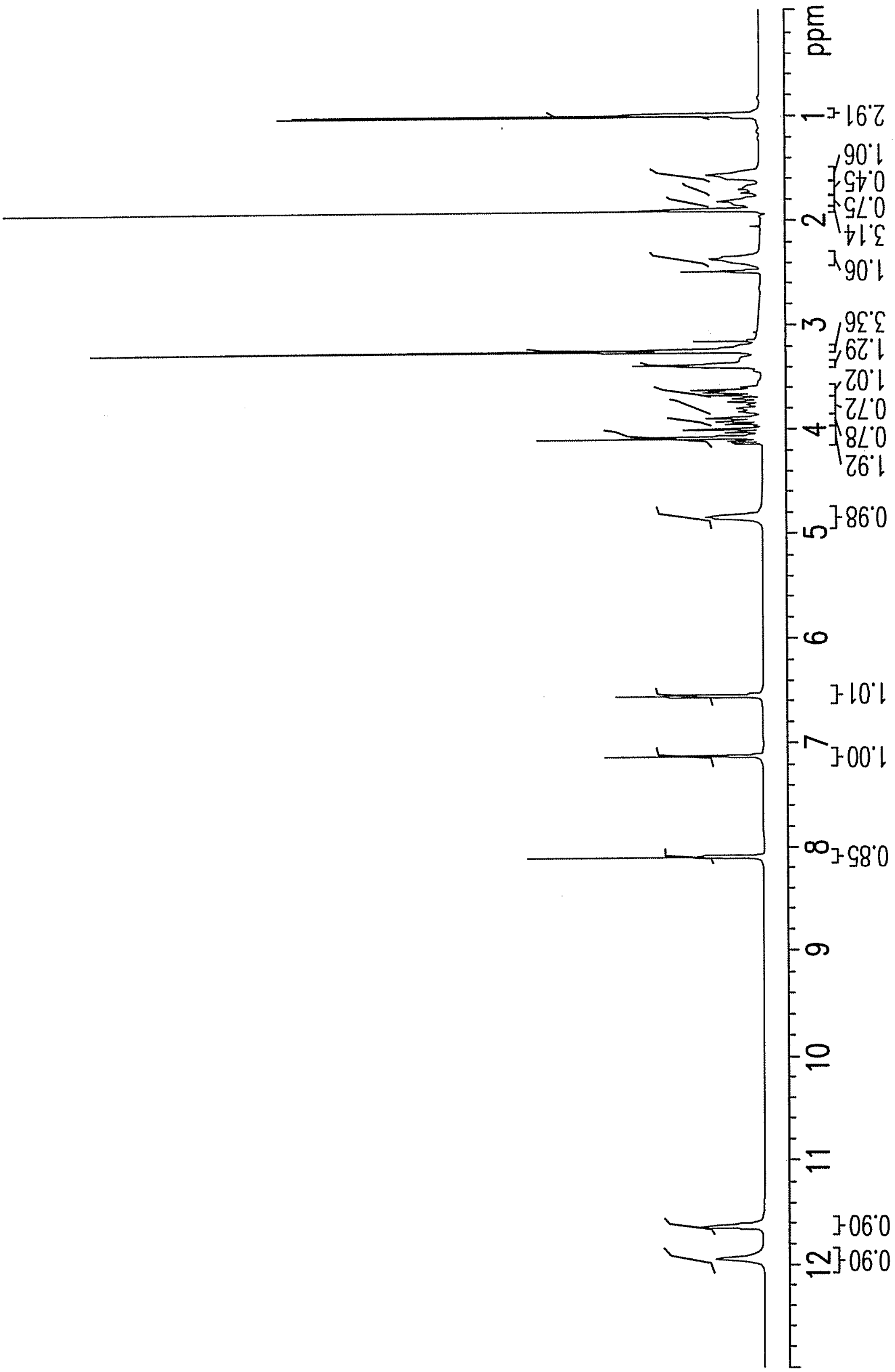


FIG. 1

A characteristic IR spectrum of Tofacitinib mono-acetate

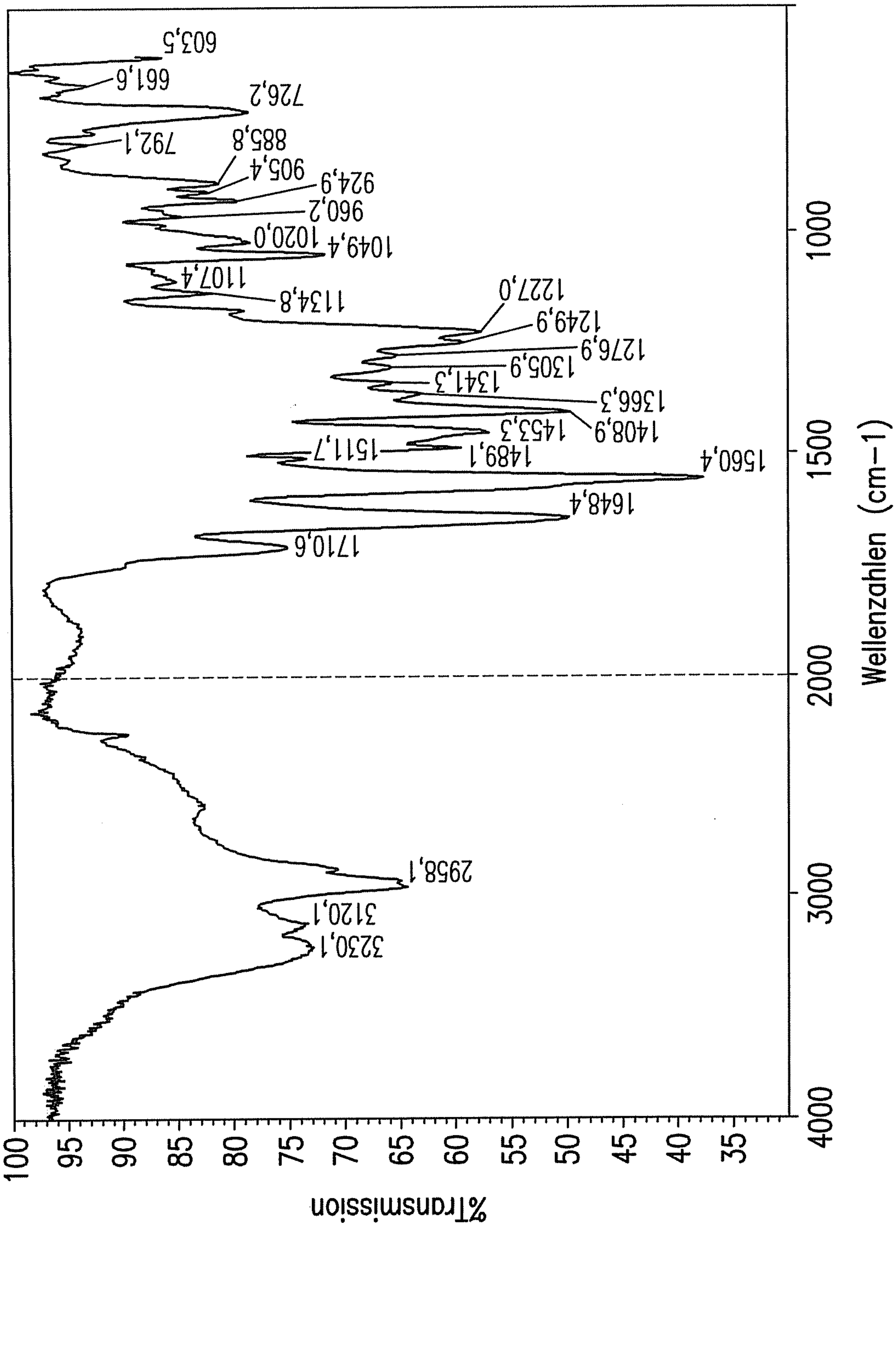


FIG.2

A characteristic X-ray powder diffractogram of Tofacitinib mono-acetate.

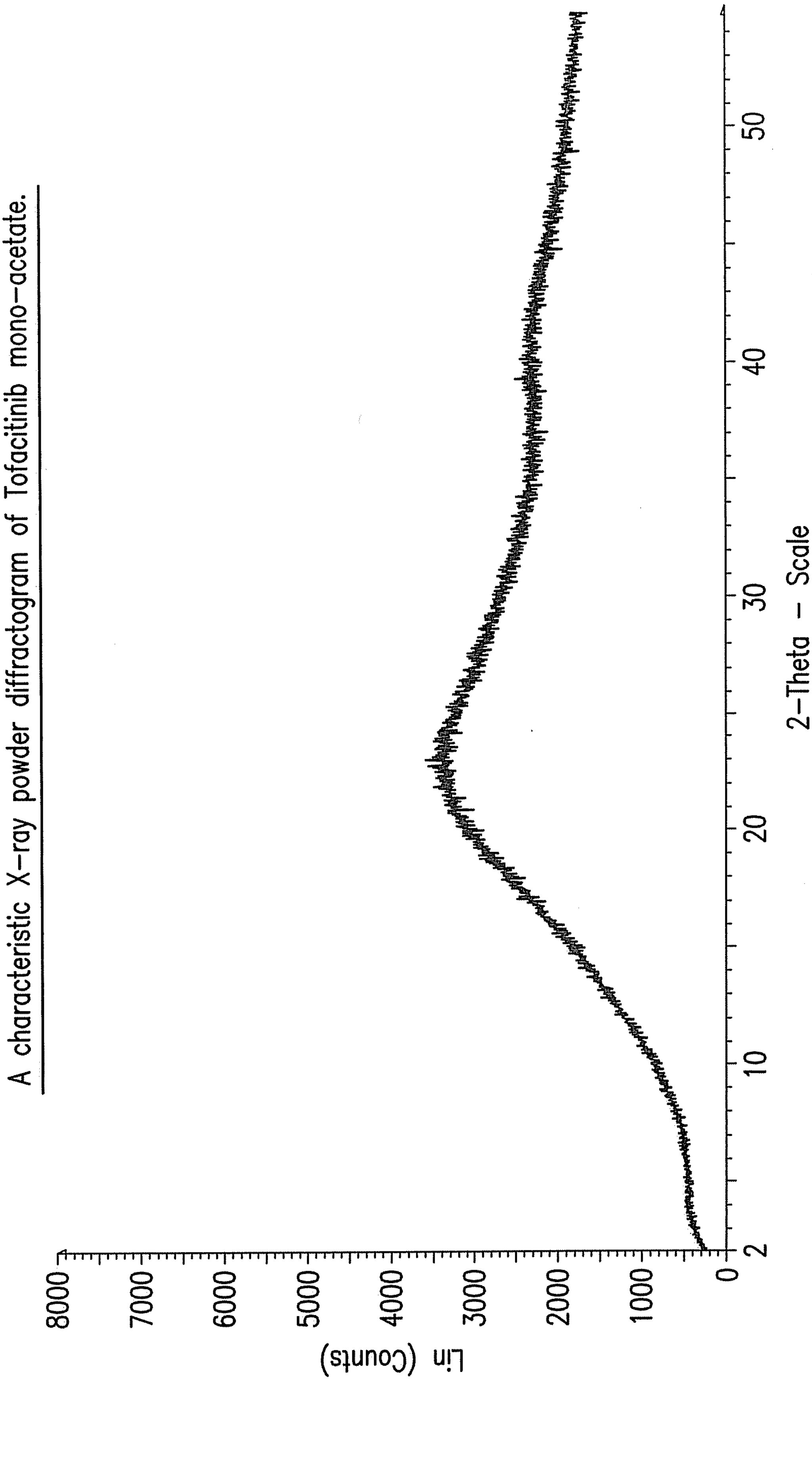


FIG. 3

A characteristic $^1$H-NMR spectrum of amporphous Tofacitinib mono-citrate.

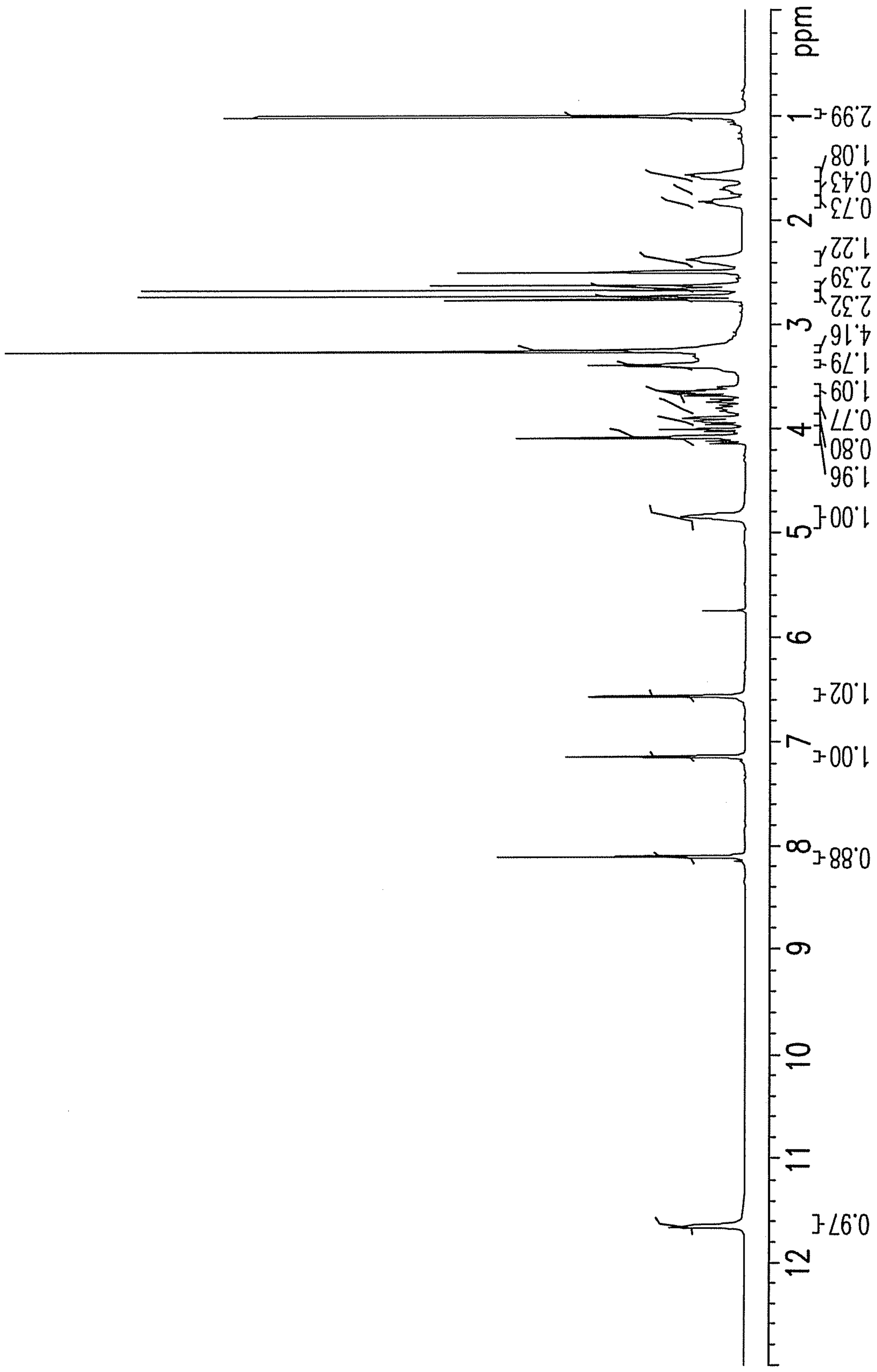

FIG. 4

A characteristic IR spectrum of amorphous Tofacitinib mono-citrate.

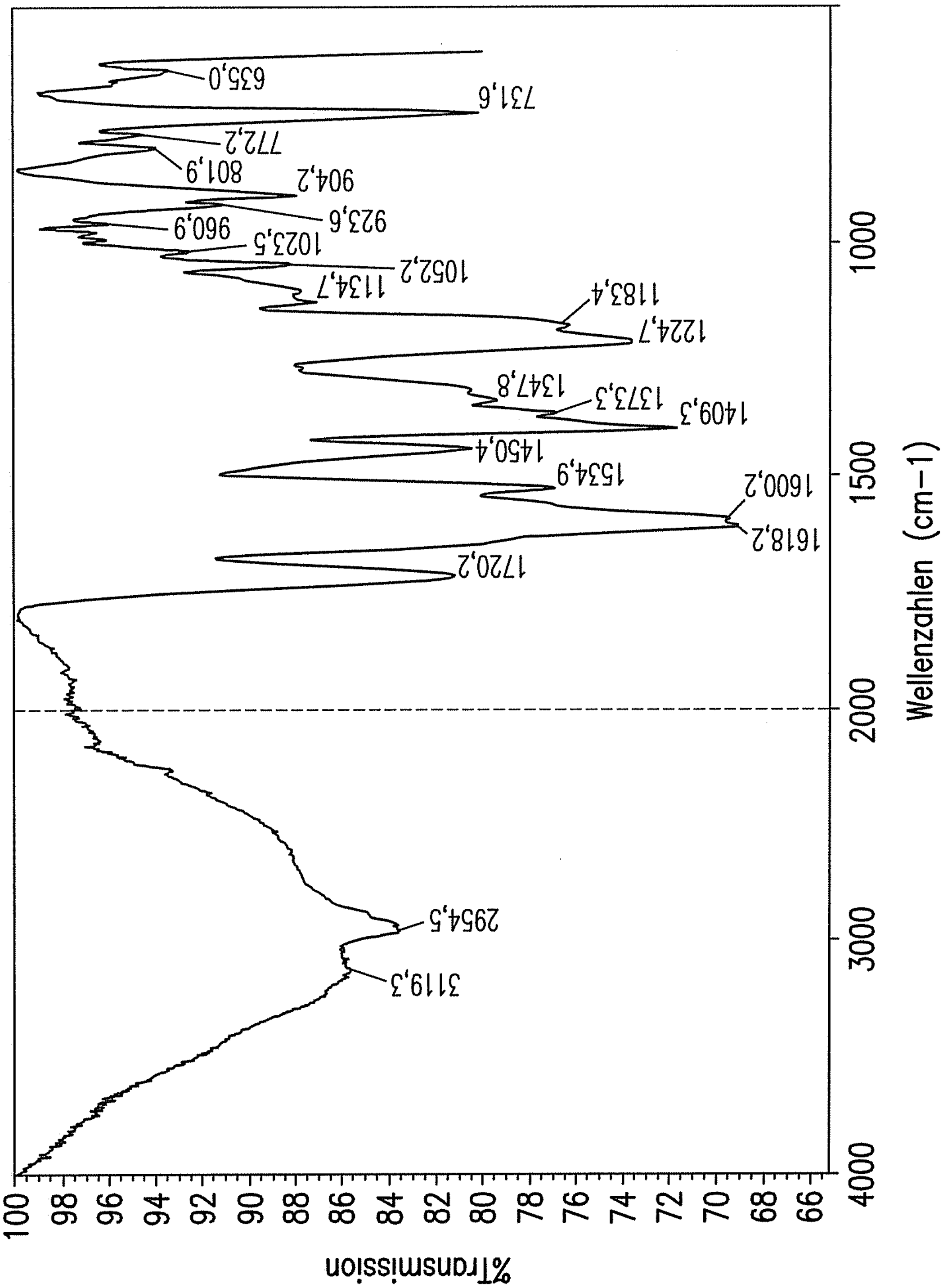


FIG.5

A characteristic X-ray powder diffractogram of a solid dispersion of Tofacitinib mono-citrate and amorphous magnesium alumino-metasilicate.

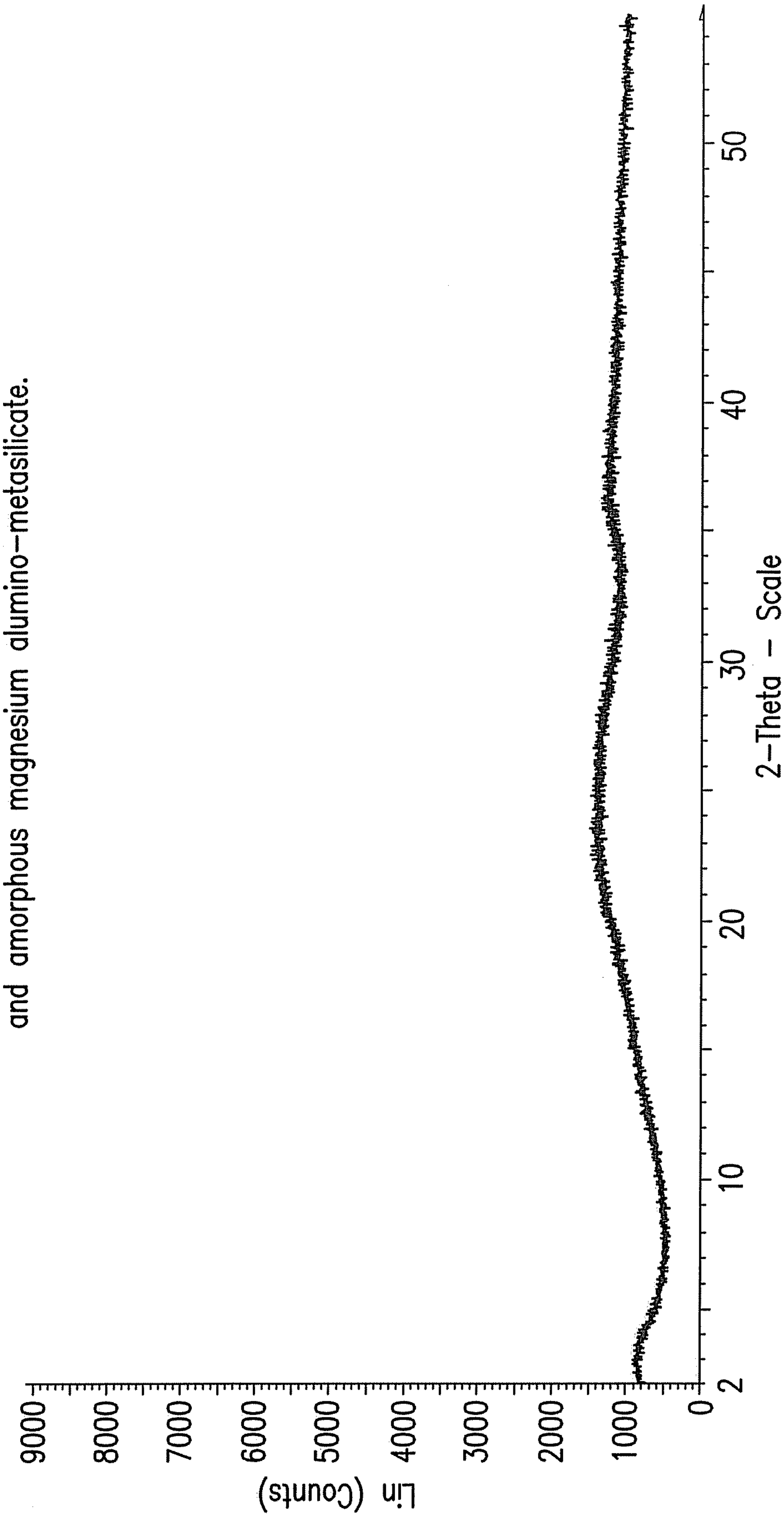


FIG.6

A characteristic X-ray powder diffractogram of a solid dispersion of Tofacitinib mono-citrate and polyvinyl pyrrolidone.

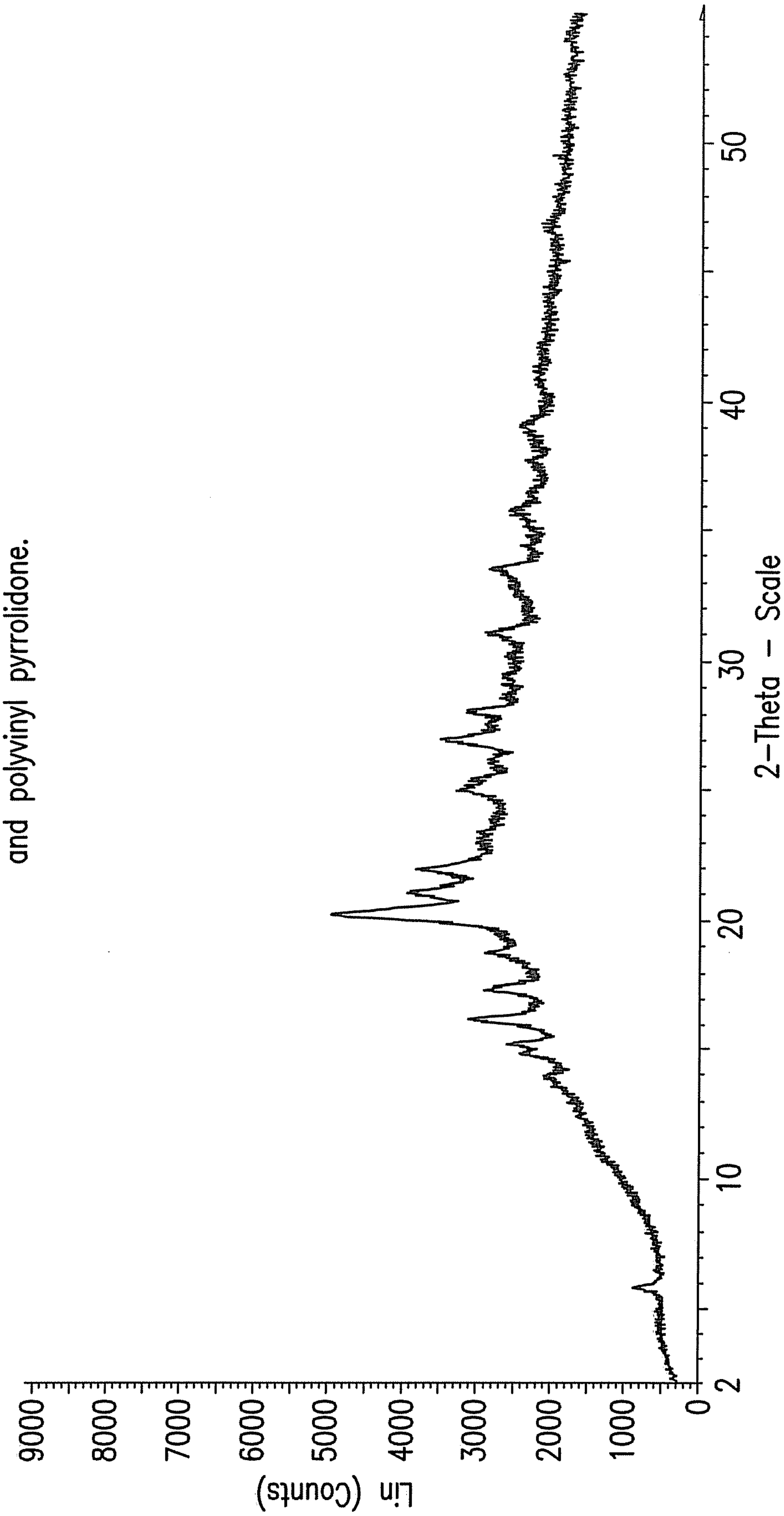


FIG. 7

A characteristic X-ray powder diffractogram of a solid dispersion of Tofacitinib mono-citrate and microcrystalline cellulose.

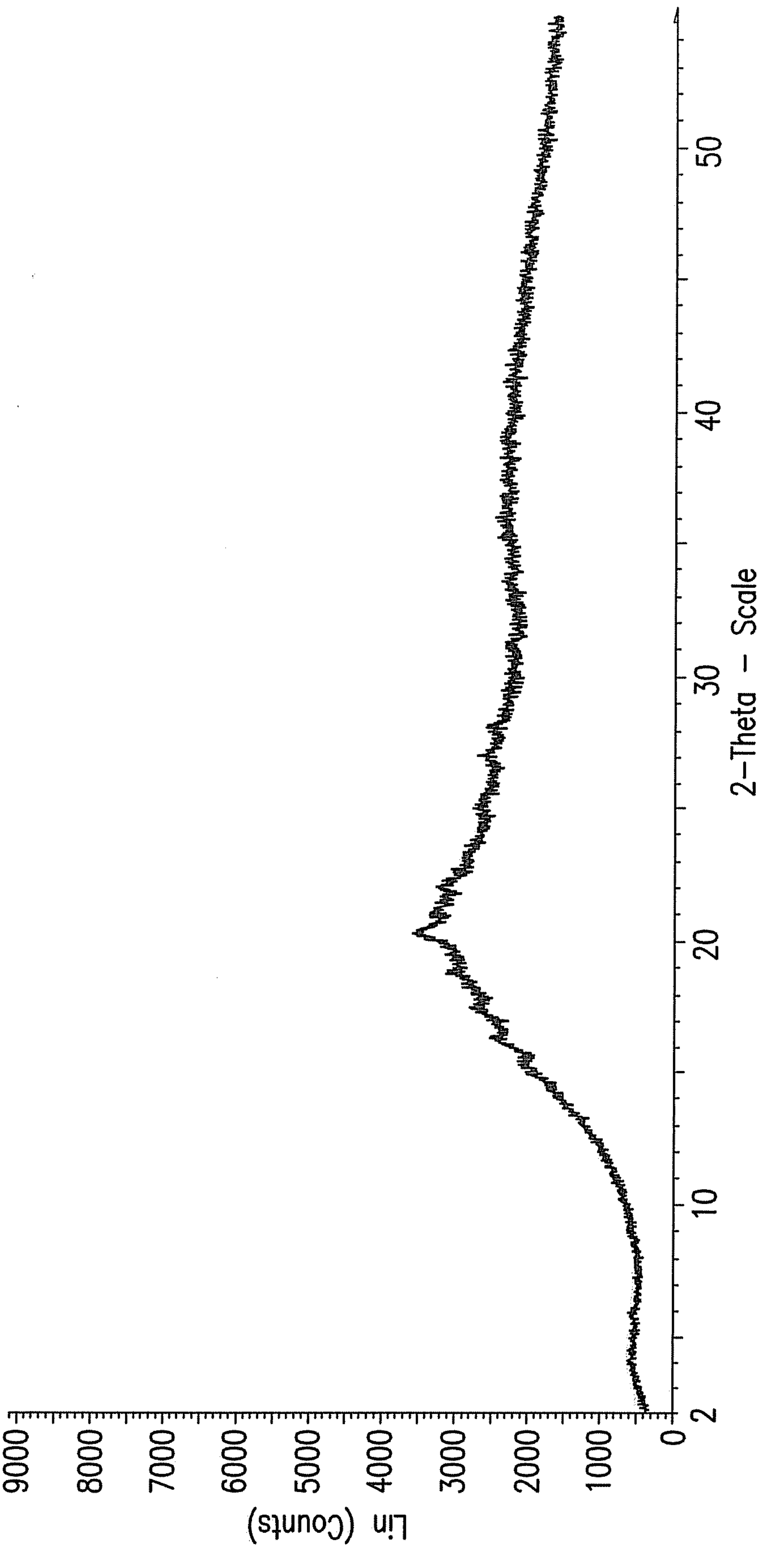


FIG.8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61468246 A
- US 61587237 A
- WO 03048126 A
- WO 01042246 A
- WO 02096909 A
- WO 03048162 A


### Non-patent literature cited in the description


- *Org. Lett.,* 2009, vol. 11 (9